# EUROPEAN PATENT SPECIFICATION

(11) **EP 1 928 928 B1**
(45) Date of publication and mention of the grant of the patent: **21.02.2018**
(21) Application number: 06793066.9
(22) Date of filing: 29.08.2006
(51) Int. Cl.: C08G 18/76, C07C 263/10, C07C 263/20, C08G 18/02

(54) **METHOD FOR THE PRODUCTION OF POLYISOCYANATES**
VERFAHREN ZUR HERSTELLUNG VON POLYISOCYANATEN
PROCÉDÉ DE PRÉPARATION DE POLYISOCYANATE

(30) Priority: 22.09.2005 EP 05108750
(43) Date of publication of application: 11.06.2008
(73) Proprietor: HUNTSMAN INTERNATIONAL LLC, Salt Lake City, Utah 84108 (US)
(72) Inventor: CARR, Robert Henry, B-3060 Bertem (BE); KOOLE, Johannes Lodewijk, B-3010 Kessel-Lo (BE); MULLER, Peter, NL-3223 SM Hellevoetsluis (NL)
(74) Representative: Van den Broeck, Kristel Alice
(86) International application number: PCT/EP2006/065786
(87) International publication number: WO 2007/039362

(56) References cited:
- WO-A-96/16028
- WO-A-2004/058689
- GB-A- 1 133 668
- US-A- 3 287 387
- Anonymous: "55 Gal Distillation Recyclers - Becca INC", , 4 May 2016 (2016-05-04), XP055270532, Retrieved from the Internet: URL:http://www.beccainc.com/products/solve nt-distillation-recycling-equipment/55-gal -distillation-recyclers/ [retrieved on 2016-05-04]

## Description

The present invention relates to a process for manufacturing non-distillable polyisocyanates such as those of the diphenyl methane series (polymeric MDI hereafter p-MDI), involving the removal of certain contaminants.

In the context of the present invention, the term polyisocyanates also includes diisocyanates as a sub-set, such as 4,4', 2,4' and 2,2'-MDI isomers and their mixtures. These are frequently produced by distillation from the polymeric mixture which can not be entirely purified by distillation. The benefits of the invention also apply to the range of prepolymers, uretonimine-modified variants, allophanate-modified variants, etc. well-known in the industry, which are subsequently produced from the purified polyisocyanates which are described specifically here.

Polyisocyanates find many applications such as in the production of polyurethane foams. Polyurethane foams are prepared by reacting polyisocyanates with polyfunctional isocyanate-reactive compounds such as polyols and polyamines, optionally in the presence of blowing agents, catalysts and other auxiliaries. Such polyurethane foams can, for example, be used as insulation material in the building industry or as cushioning material for furniture or automotive industry.

In the automotive industry, a recognized problem has been the formation of volatile condensate or "fog" on the interior and windshield of the automobile. This residue is unsightly, and may impair the vision of the driver under certain circumstances.

In response to the fogging problem, the automotive industry has developed a standard test to quantify the fogging characteristics of materials used in automotive interiors (DIN 75 201, determination of the fogging behavior of materials for interior automotive trim). The content of volatile organic compounds (VOC) is also a subject of analytical determinations (Volkswagen central standard 55 031, Daimler Chrysler PB VWT 709). The Daimler-Chrysler method requires the assignment of the emissions to individual chemical compounds in addition to the quantitative determination of the VOC and fog value. The emitted compounds can also contribute to the perceived odor of finished products.

It should be noted that the "fogging" problem is not unique to the automotive industry. Anti-fogging foams have applications in other areas where dirt and condensate residue would have a deleterious effect. Such applications would include, for example, electronics or semiconductor manufacturing facilities, electronics packaging, clean rooms, and medical device applications.

VOC and fog problems can have many origins and there have therefore been many attempts to reduce contributions to VOC and fog levels in different ways. For example, US 6423758 describes a cellular foam composition having anti-fogging characteristics and the method of making the same. US 5958993 describes the use of anti-fogging flame retardants; US 6306918 describes the use of an amine catalyst having a primary hydroxyl group such that it reacts into the polymer matrix; US 6458860 describes a catalyst system useful for providing polyurethane foam products which exhibit low fogging characteristics. US 5770659 describes polyetherester resins for low-VOC formulations.

As well as polyurethane (PU) products, low fog and low VOC specifications can exist for polyisocyanurate (PIR) foams, polyurea products, composite materials (PU and/or PIR with other materials) and products where isocyanates are used as adhesive or binder (for example, replacing urea-formaldehyde in wood panel products or as a binder for so-called "rubber-crumb" surfaces such as children's playgrounds). These same volatile contaminants which can contribute to VOC and fogging can also impart odor to finished products and their elimination or reduction can, therefore, also have beneficial effects on customer perceptions and the work environment of production employees.

It has now surprisingly been found that volatile, aromatic, non-isocyanate-group-containing (hereafter non-NCO) contaminants contribute to VOC and fog problems in products derived from polyisocyanates. In the context of this invention, the contaminants are compounds other than the normally expected compounds present in polyisocyanates such as residual levels of reactants, by-products, etc. of the phosgenation process. For clarity, the contaminants considered here do not include residual levels of phosgene, the chosen phosgenation process solvent (e.g. mono-chlorobenzene), by-product HCl or unconverted amine reactant.

The purpose of the current invention is a process to eliminate or greatly reduce the level of VOC and fogging contaminants in the final product by removing a contaminant-enriched solvent stream from the polyisocyanate production process equipment.

This invention differs significantly from prior art cases such as WO 2004/058689 and WO 96/16028 which include a process stage where the entire recycling process solvent is subjected to purification by a fractional distillation with, presumably, removal of contaminants. Fractional distillation of the entire process solvent recycle in such large-scale industrial processes as are used to manufacture MDI polyisocyanates on a commercial scale is a significant economic and technological cost (in terms of energy use, process equipment scale and cost, together with operational and safety issues). Thus, significant economic and technological benefits can be achieved surprisingly by means of treatment of only a part of the process solvent as described in the current invention.

The non-NCO contaminant compounds to be removed according to the present invention include but are not limited to: nitrobenzene and dinitrobenzene (present, for example, because of residual levels in the aniline used to make the aniline-formaldehyde condensates subsequently converted to methylene diphenyl diisocyanate & higher oligomers - MDI and polymeric MDI); nitrotoluene and dinitrotoluene isomers (present, for example, because of residual levels in the diaminotoluene subsequently converted to toluene diisocyanate - TDI); dichlorobenzene isomers (hereafter DCB's) (present, for example, because of reaction of chlorine with monochlorobenzene, a phosgenation solvent); chlorotoluene isomers, bromobenzene, bromotoluene isomers, bromochlorobenzene isomers, bromochlorotoluene isomers (present, for example, because of reaction of chlorine and/or bromine with other compounds present in the production plant). Contaminants which have volatilities similar to that of the phosgenation solvent have been dealt with by treatment of the separated solvent. GB 848986 discloses subjecting the used solvent to a heat treatment at 150-200°C to cause precipitation of contaminants which are then separated by filtration or centrifuging. The contaminants which are removed include residual isocyanate compounds. The thermal purification treatment may be associated with a treatment with about 2% of a substance containing -OH or -NH groups capable of reacting with the isocyanate compounds remaining in the used solvent and converting them into insoluble compounds. US 4405527 describes a process for the preparation of polyisocyanates in the presence of solvents, in which the solvent is freed from traces of compounds containing isocyanate groups before it is reused. The solvent is treated with compounds containing isocyanate reactive hydrogen atoms, such as alcohols or amines, to convert the readily volatile isocyanates into reaction products containing urethane or urea groups. The treated solvent is then separated from these reaction products by distillation. In US 4745216 the solvent to be freed from traces of isocyanate and to be reused is treated with certain polymers and then separated mechanically (e.g. by decanting or filtration) from these polymers. The polymers employed are crosslinked polymers which are insoluble in the solvent and contain at least one functional group selected from primary alcoholic hydroxyl groups, secondary alcoholic hydroxyl groups, primary amino groups and secondary amino groups.

None of the above mentioned prior art provides an effective means of dealing with the volatile, aromatic, non-isocyanate- group-containing (non-NCO) contaminants which are the object of this invention and which, if retained in the polyisocyanate product, could contribute to the odor, VOC or fog from derived polyurethane or other products.

Thus, there remains a need for a process for eliminating or reducing the levels of non-NCO contaminants in polyurethane foams and other products based on or incorporating polyisocyanates which would otherwise contribute to fogging or VOC levels.

The process of the present invention can be applied in the production of any type of organic polyisocyanate. Particular preference goes to the aromatic polyisocyanates such as diphenylmethane diisocyanate in the form of its 2,4'-, 2,2'- and 4,4'-isomers and mixtures thereof, the mixtures of diphenylmethane diisocyanates (MDI) and oligomers thereof known in the art as "crude" or polymeric MDI (polymethylene polyphenylene polyisocyanates) having an isocyanate functionality of greater than 2, and, generally, those isocyanate products which can not be distilled.

Optionally, the invention can also be applied to toluene diisocyanate in the form of its 2,4- and 2,6-isomers and mixtures thereof, 1,5-naphthalene diisocyanate and 1,4-diisocyanatobenzene. Other suitable organic polyisocyanates, which may be mentioned, include the aliphatic diisocyanates such as isophorone diisocyanate, 1,6-diisocyanatohexane and 4,4'-diisocyanatodicyclohexylmethane. However, being relatively low molecular weight pure compounds, these and other relatively volatile isocyanate products can conventionally be purified directly by fractional distillation.

Most preferably the present process is applied in the production of polyisocyanates of the diphenyl methane series. In such a case the low molecular weight non-NCO contaminants are primarily, but not exclusively, bromobenzene, bromotoluene, chlorotoluene, benzonitrile, dichlorobenzene isomers, bromochlorobenzene isomers, chloroisopropyl benzene isomers, dichlorotoluene isomers, trichlorobenzene isomers, nitrobenzene, dinitrobenzene, nitrotoluene, dinitrotoluene, chloronitrobenzene isomers, chloronitrotoluene isomers and trichlorotoluene isomers.

The present invention relates to a process for the preparation of polyisocyanates by the reaction of polyamines from which the polyisocyanates are derived preferably as solutions in an inert solvent with phosgene optionally as a solution in an inert solvent by single stage or multi-stage phosgenation reaction or any variation known to the art, in batch, continuous or semi-continuous modes, at atmospheric pressure or above. After completion of the phosgenation reaction, the reaction mixture is distilled. The solvent is then treated to concentrate traces of non-NCO contaminants and largely reused for the preparation of amine solution and/or phosgene solution.

In this process only part of the solvent is treated.

Particular embodiments of the present invention include:
(i) stepwise distillation of the phosgenation reaction mixture to prepare a solvent stream particularly enriched in non-NCO contaminants;
(ii) further partial treatment of the solvent removed from the phosgenation reaction mixture, either by further distillation or any other known method, to prepare a solvent stream particularly enriched in non-NCO contaminants;
(iii) return of the solvent which has been treated to remove non-NCO contaminants to another suitable part of the polyisocyanate production plant, for example, a phosgenation reactor or the solvent distillation vessel;
(iv) removal of the solvent enriched in non-NCO contaminants from the production process for further treatment or destruction by known methods e.g. incineration;
(v) operation of any or all of the above described processes or sub-units of operation in either batch, continuous or semi-continuous modes at atmospheric pressure or above.

These embodiments may also be combined with a process or processes for dealing with volatile, isocyanate-group-containing compounds, for example, trimerisation of phenyl isocyanate and similar compounds.

It is to be understood that the above mentioned embodiments are described solely for purposes of illustration and that combinations of these or similar variations not specifically described are also included within the present invention.

The invention is thus related to a process for preparation of polyisocyanates containing less than 50 ppm of total volatile, non-isocyanate-group-containing contaminants, composed of the following individual stages:
(a) reaction of (i) solutions of the polyamine(s) underlying the polyisocyanate(s) in an inert solvent with (ii) a solution of phosgene optionally in an inert solvent in a single stage or multi-stage reaction of phosgenation;
(b) separation of the excess phosgene and of the hydrogen chloride formed from the liquid reaction mixture obtained by (a);
(c) separation of the solvent together with readily volatile compounds from the solution obtained in (b) by evaporation; and recovery of the product of the process as evaporation residue which is optionally subjected to a further process of distillation;
(d) recovery of a solvent containing volatile compound(s) by condensation of the vapors obtained in (c) and reuse of part of the condensate for the preparation of amine solution (i) and optionally of another part of the condensate for the preparation of phosgene solution (ii);
(e) removal of a solvent stream enriched in volatile, aromatic, non-NCO contaminants from the polyisocyanate production process for further treatment or destruction.

The phosgenation reaction is carried out in any known manner, using solutions of polyamines in inert solvents and phosgene optionally as solution in inert solvents. In the process of the present invention, this phosgenation reaction may be carried out either in one stage or in several stages. For example, phosgenation may be carried out by forming suspensions of carbamic acid chlorides at low temperatures and then converting these suspensions into polyisocyanate solutions at elevated temperatures ("cold/hot, two-stage phosgenation").

Alternatively, special mixing devices may be employed to enable rapid mixing of the amine and phosgene streams so that side reactions are minimised and the preferred phosgenation reaction predominates. Many variations of such a process are known. Particularly suitable polyamine starting materials are the technically important polyamines such as 2,4'-, 2,2'- and 4,4'-diaminodiphenyl methane and their mixtures with higher homologues (known as "polyamine mixtures of the diphenyl methane series") which may be obtained in known manner by aniline/formaldehyde condensation. Other starting materials can include hexamethylene diamine; 2,4- and/or 2,6-diamino toluene; 1,5-diaminonaphthalene; 1-amino-3,3,5-trimethyl-5-aminomethyl-cyclohexane (isophorone diamine); tris-(isocyanatophenyl)-methane and perhydrogenated diaminodiphenyl methanes and their mixtures with higher homologues.

In the process of the present invention, the amine starting materials such as those mentioned as examples above may be used in the form of 3 to 50 wt%, preferably 5 to 40 wt% solutions in inert solvents. The phosgene required for the phosgenation reaction is generally used in the form of a 10 to 60 wt%, preferably 25 to 50 wt% solution in inert solvents or, optionally, without solvent.

Suitable inert solvents both for the polyamine and for phosgene are known to those in the art. Exemplary solvents are chlorinated aryl and alkylaryl hydrocarbons especially monochlorobenzene (MCB). Other solvents can be used with suitable process variations and include o-dichlorobenzene, trichlorobenzene and the corresponding toluene, xylene, methylbenzene and naphthalene compounds, and many others known in the art such as toluene, xylenes, nitrobenzene, ketones, and esters.

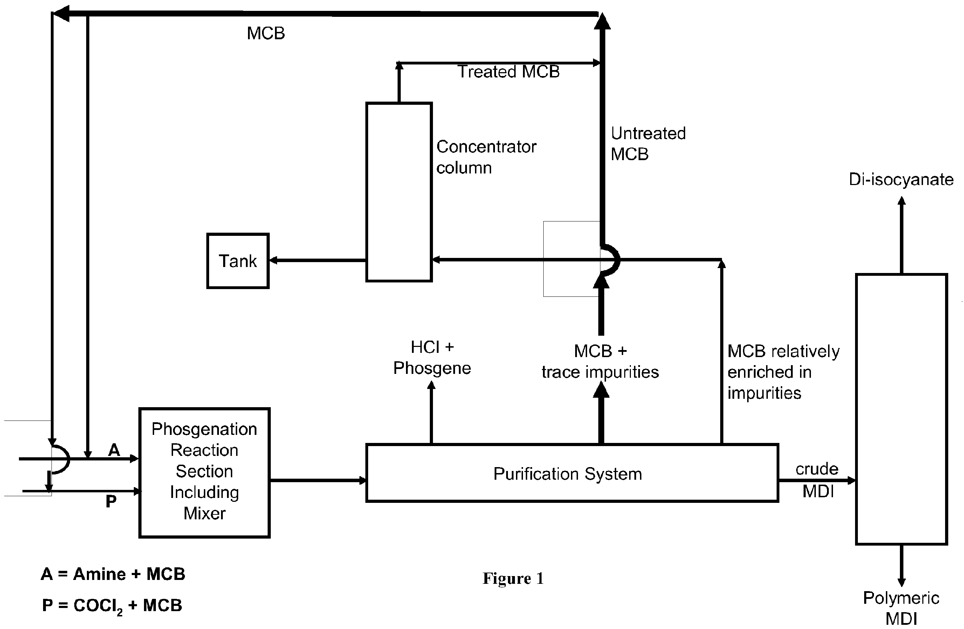

After the phosgenation has been carried out by methods known in the art, the excess phosgene and the hydrogen chloride formed are removed by methods known in the art, such as by blowing them out with inert gas or by partial distillation. The phosgenation product present in the form of a solution is then separated, either simply by evaporation or by fractional distillation, into a gaseous phase containing solvent together with volatile compounds and a liquid phase substantially made up of crude polyisocyanate. The liquid phase obtained may, if desired, be worked up by distillation in known manner if a pure polyisocyanate is to be produced. This separation of crude polyisocyanate and volatile compounds is generally carried out at a temperature of from 80 to 220°C (preferably from 120 to 190°C) at a pressure of from 10 to 4000 mbar (preferably from 100 to 3000 mbar). The vapor containing solvent together with volatile compounds is condensed to form a solvent condensate containing volatile contaminants. This may be processed further, for example, by additional fractional distillation, to give a solvent stream greatly enriched in the volatile contaminant compounds. This stream is then removed from the polyisocyanate production process for additional further processing or destruction for example by incineration. Optionally, this may include temporary storage in a tank or other suitable vessel. The further processing may be by means of on-site or off-site facilities and may be carried out by means of pipelines or transfer to transportable vessels. A schematic representation given soley for the purpose of illustration is presented as Figure 1.

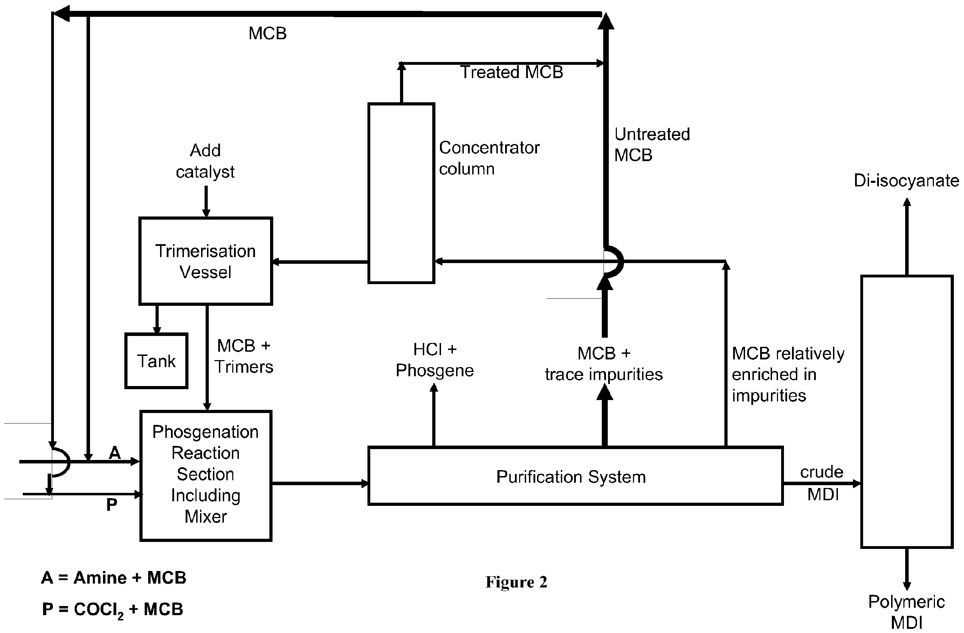

This process may optionally also be combined with a process or processes for dealing with volatile, isocyanate-group-containing compounds, for example, trimerisation of phenyl isocyanate and similar compounds. A schematic representation given soley for the purpose of illustration is presented as Figure 2.

The quality of the (monochlorobenzene) solvent, now substantially free of contaminants, can be determined by on-line analysis techniques such as spectroscopic or chromatographic techniques (Near Infra-red spectroscopy, infra-red spectroscopy, gas chromatography) in order to ensure contaminants have been removed to the required levels. For example, phenyl isocyanate, MDI, water, nitrobenzene, dichlorobenzenes can all be determined by on-line FT-IR spectroscopy. Results from on-line analysis can be used to monitor the effectiveness of the process and, if necessary, adjust aspects of the equipment control, either automatically or with manual intervention.

The relatively small quantity of solvent lost from the system together with the contaminants can be replaced by fresh solvent from storage.

By using the process of the present invention polyisocyanates are obtained that contain in total less than 50 ppm of volatile, aromatic, non-NCO-group-containing contaminants; polyisocyanates than contain no such contaminants at all are included within the invention. The content of individual volatile, aromatic, non-NCO-group-containing contaminants (e.g. p-dichlorobenzene) is generally below 10 ppm, preferably below 2 ppm and most preferably below 1 ppm.

The intent of the present invention is illustrated for example by demonstrating the correlation between one particular volatile aromatic non-NCO contaminant compound in polyisocyanate and the VOC level in polyurethane foam. In order to determine what level of pDCB in polyisocyanate could be detected in the VOC test, four conventional flexible foam samples were prepared using polyisocyanate doped specially with para-dichlorobenzene (pDCB). Two reference foam samples were prepared from un-doped polyisocyanate. The pDCB released from the derived foam was measured in the standard Daimler-Chrysler VOC test. Each foam was sampled & analysed twice. Details are given in the following table.

| Foam | pDCB added to isocyanate | Isocyanate to polyol ratio | pDCB added to foam | Found #A | Found #B | Average Found |
|---|---|---|---|---|---|---|
| | ppm | in foam | microg/g | microg/g | microg/g | microg/g |
| 1 | 0 | 50/100 | 0 | 20.8 | 19.0 | 19.9 |
| 2 | 504 | 50/100 | 168 | 97.6 | 97.9 | 97.8 |
| 3 | 1024 | 50/100 | 341 | 202.1 | 193.5 | 197.8 |
| 4 | 504 | 50/100 | 181 | 130.1 | 133.8 | 132.0 |
| 5 | 1024 | 50/100 | 368 | 260.7 | 255.4 | 258.1 |
| 6 | 0 | 50/100 | 0 | 30.2 | 32.8 | 31.5 |

The fact that significantly less pDCB was measured than was added to the polyisocyanate is easily explainable due to losses of this relatively volatile compound during the foaming process. Applying a simple linear fit to the data indicates that the original polyisocyanate sample contained about 20 ppm pDCB. The signal:noise ratio for the analytical method (gas chromatography with mass spectrometric detection) is such that an order of magnitude lower detection is easily attainable. Thus, polyisocyanate with less than 2 ppm, preferably less than 1 ppm, of pDCB is desirable from the production process in order to reduce the VOC of this specific contaminant from the derived foam. The degree of concentration of contaminants in the separated phosgenation solvent and the rate of removal of material from the production process in order to achieve the required level in polyisocyanate product can be determined in operation by those skilled in the art.

It is to be understood that the above example is provided only as an illustration of the principle of the invention. Similar characterisation can be carried out for any target non-NCO contaminant by those skilled in the art.

## Claims

1. Process for preparing polyisocyanates containing less than 50 ppm of total volatile, aromatic, non-isocyanate- group-containing contaminants, the process involving
a) reacting solutions of (i) polyamine(s) underlying the polyisocyanate(s) in an inert solvent with (ii) a solution of phosgene optionally in an inert solvent in a single stage or multi-stage reaction of phosgenation;
b) separation of the excess phosgene and of the hydrogen chloride formed from the liquid reaction mixture obtained by a);
c) separation of the solvent together with readily volatile compounds from the solution obtained in b) by evaporation; and recovery of the product as evaporation residue which is then optionally subjected to a further process of distillation;
d) recovery of a solvent containing volatile compound(s) by condensation of the vapors obtained in c) and reuse of part of the condensate for the preparation of amine solution (i) and optionally of another part of the condensate for the preparation of phosgene solution (ii);
e) removal of a solvent stream enriched in volatile, aromatic, non-NCO contaminants from the polyisocyanate production process for further treatment or destruction.

2. Process according to claim 1 wherein before reuse of part of the condensate in step d) also volatile, aromatic, isocyanate-group-containing compounds are removed from the solvent.

3. Process according to claim 2 wherein said volatile, aromatic, isocyanate-group-containing compounds are removed by trimerising them.

4. Process according to any one of the preceding claims, wherein said contaminants are selected from the group consisting of nitrobenzene, dinitrobenzene, nitrotoluene and dinitrotoluene isomers, dichlorobenzene isomers, trichlorobenzene isomers, chlorotoluene isomers, bromobenzene, bromotoluene isomers, bromochlorobenzene isomers, bromochlorotoluene isomers, benzonitrile, chloroisopropylbenzene isomers, dichlorotoluene isomers, chloronitrobenzene isomers, chloronitrotoluene isomers and trichlorotoluene isomers.

5. Process according to any one of the claims 1 to 4, wherein the polyisocyanate is of the diphenylmethane series.

6. Process according to claim 5, wherein the contaminant comprises p-dichlorobenzene.

7. Process according to any one of the preceding claims wherein the level of an individual contaminant is less than 2 ppm.

8. Process according to any one of the preceding claims wherein the level of an individual contaminant is less than 1 ppm.

## Patentansprüche

1. Verfahren zur Herstellung von Polyisocyanaten, die weniger als 50 ppm an flüchtigen, aromatischen, nicht Isocyanatgruppen enthaltenden Verunreinigungen insgesamt enthalten, wobei das Verfahren folgendes beinhaltet
a) Umsetzen von Lösungen von (i) Polyamin(en), die dem/den Polyisocyanat(en) zugrunde liegen, in einem inerten Lösungsmittel mit (ii) einer Lösung von Phosgen, gegebenenfalls in einem inerten Lösungsmittel, in einer einstufigen oder mehrstufigen Reaktion der Phosgenierung;
b) Abtrennung des überschüssigen Phosgens und des gebildeten Chlorwasserstoffs von der in a) erhaltenen flüssigen Reaktionsmischung;
c) Abtrennung des Lösungsmittels zusammen mit leicht flüchtigen Verbindungen aus der in b) erhaltenen Lösung durch Verdampfung; und Gewinnung des Produkts als Verdampfungsrückstand, der dann gegebenenfalls einem weiteren Destillationsprozess unterworfen wird;
d) Gewinnung eines eine oder mehrere flüchtige Verbindung(en) enthaltenden Lösungsmittels durch Kondensation der in c) erhaltenen Dämpfe und Wiederverwendung eines Teils des Kondensats zur Herstellung von Aminlösung (i) und gegebenenfalls eines anderen Teils des Kondensats zur Herstellung von Phosgenlösung (ii);
e) Entfernung eines Lösungsmittelstroms, der mit flüchtigen, aromatischen, nicht-NCO-Verunreinigungen aus dem Polyisocyanat-Herstellungsverfahren angereichert ist, zur weiteren Behandlung oder Beseitigung.

2. Verfahren nach Anspruch 1, wobei vor Wiederverwendung eines Teils des Kondensats in Schritt d) auch flüchtige, aromatische, Isocyanatgruppen enthaltende Verbindungen aus dem Lösungsmittel entfernt werden.

3. Verfahren nach Anspruch 2, wobei die flüchtigen, aromatischen Isocyanatgruppen enthaltenden Verbindungen durch ihre Trimerisierung entfernt werden.

4. Verfahren nach irgendeinem der vorhergehenden Ansprüche, wobei die Verunreinigungen ausgewählt sind aus der Gruppe bestehend aus Nitrobenzol, Dinitrobenzol, Nitrotoluol und Dinitrotoluol-Isomeren, Dichlorbenzol-Isomeren, Trichlorbenzol-Isomeren, Chlortoluol-Isomeren, Brombenzol, Bromtoluol-Isomeren, Bromchlorbenzol-Isomeren, Bromchlortoluol-Isomeren, Benzonitril, Chlorisopropylbenzol-Isomeren, Dichlortoluol-Isomeren, Chlornitrobenzol-Isomeren, Chlornitrotoluol-Isomeren und Trichlortoluol-Isomeren.

5. Verfahren nach irgendeinem der Ansprüche 1 bis 4, wobei das Polyisocyanat aus der Diphenylmethanreihe ist.

6. Verfahren nach Anspruch 5, wobei die Verunreinigung p-Dichlorbenzol umfasst.

7. Verfahren nach irgendeinem der vorhergehenden Ansprüche, wobei der Gehalt einer individuellen Verunreinigung weniger als 2 ppm beträgt.

8. Verfahren nach irgendeinem der vorhergehenden Ansprüche, wobei der Gehalt einer individuellen Verunreinigung weniger als 1 ppm beträgt.

## Revendications

1. Procédé de préparation de polyisocyanates contenant moins de 50 ppm de contaminants contenant un groupe non isocyanate aromatiques, volatils, totaux, le procédé impliquant :
a) la réaction de solutions de (i) polyamine(s) sous-jacente(s) au(x) polyisocyanate(s) dans un solvant inerte avec (ii) une solution de phosgène éventuellement dans un solvant inerte dans une réaction de phosgénation à simple étape ou multi-étapes ;
b) la séparation de l'excès de phosgène et du chlorure d'hydrogène formé à partir du mélange réactionnel liquide obtenu par a) ;
c) la séparation du solvant conjointement avec les composés facilement volatils de la solution obtenue dans b) par évaporation ; et la récupération du produit comme résidu d'évaporation qui est ensuite éventuellement soumis à un autre processus de distillation ;
d) la récupération d'un solvant contenant le(s) composé(s) volatil(s) par condensation des vapeurs obtenues dans c) et la réutilisation d'une partie du condensat pour la préparation d'une solution d'amine (i) et éventuellement d'une autre partie du condensat pour la préparation d'une solution de phosgène (ii) ;
e) l'enlèvement d'un courant de solvant enrichi en contaminants non NCO, aromatiques, volatils, du procédé de production de polyisocyanate pour un autre traitement ou destruction.

2. Procédé selon la revendication 1, dans lequel, avant la réutilisation d'une partie du condensat dans l'étape d), des composés contenant un groupe isocyanate, aromatiques, volatils, sont aussi enlevés du solvant.

3. Procédé selon la revendication 2, dans lequel lesdits composés contenant un groupe isocyanate, aromatiques, volatils, sont enlevés en les trimérisant.

4. Procédé selon l'une quelconque des revendications précédentes, dans lequel lesdits contaminants sont choisis dans le groupe constitué par les isomères de nitrobenzène, de dinitrobenzène, de nitrotoluène et de dinitrotoluène, les isomères de dichlorobenzène, les isomères de trichloro-benzène, les isomères de chlorotoluène, le bromobenzène, les isomères de bromotoluène, les isomères de bromochloro-benzène, les isomères de bromochlorotoluène, le benzo-nitrile, les isomères de chloroisopropylbenzène, les isomères de dichlorotoluène, les isomères de chloronitro-benzène, les isomères de chloronitrotoluène et les isomères de trichlorotoluène.

5. Procédé selon l'une quelconque des revendications 1 à 4, dans lequel le polyisocyanate est de la série du diphénylméthane.

6. Procédé selon la revendication 5, dans lequel le contaminant comprend du p-dichlorobenzène.

7. Procédé selon l'une quelconque des revendications précédentes, dans lequel le niveau d'un contaminant individuel est de moins de 2 ppm.

8. Procédé selon l'une quelconque des revendications précédentes, dans lequel le niveau d'un contaminant individuel est de moins de 1 ppm.
